(19) Europäisches Patentamt
European Patent Office
Office européen des brevets

(11) EP 0 600 045 B1

(12) **EUROPÄISCHE PATENTSCHRIFT**

(45) Veröffentlichungstag und Bekanntmachung des Hinweises auf die Patenterteilung:
**24.01.1996 Patentblatt 1996/04**

(21) Anmeldenummer: **93906610.6**

(22) Anmeldetag: **25.03.1993**

(51) Int. Cl.$^6$: **A61K 7/13**, C09B 29/08

(86) Internationale Anmeldenummer: **PCT/EP93/00722**

(87) Internationale Veröffentlichungsnummer: **WO 93/25182 (23.12.1993 Gazette 1993/30)**

(54) **HAARFÄRBEMITTEL**
HAIR DYE
COLORANT CAPILLAIRE

(84) Benannte Vertragsstaaten:
**DE ES FR GB IT**

(30) Priorität: **17.06.1992 DE 4219738**

(43) Veröffentlichungstag der Anmeldung:
**08.06.1994 Patentblatt 1994/23**

(73) Patentinhaber: **Wella Aktiengesellschaft**
**D-64295 Darmstadt (DE)**

(72) Erfinder: **BRAUN, Hans, Jürgen**
**D-3182 Ueberstorf (CH)**

(56) Entgegenhaltungen:
**FR-A- 2 282 860** **GB-A- 2 165 257**

- **INDIAN J. FIBRE TEXT. RES. Bd. 15, Juni 1990, Seiten 76 - 80 RANGNEKAR D.W. ET AL 'SYNTHESIS AND DYEING CHARACTERISTICS OF MONO- AND BIS-AZO-4-HYDROXY-1-METHYL-2-QUINOLONES'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 600 045 B1

## Beschreibung

Gegenstand der Erfindung ist ein Mittel zum Färben von Haaren mit einem Gehalt an einem 4'-Amino-2-halogen-4-[bis-(β-hydroxyethyl)amino]-azobenzol.

Für das Färben von Haaren gewinnen, neben den Oxidationshaarfarbstoffen, die durch oxidative Kupplung einer oder mehrerer Entwicklerkomponenten untereinander gebildet werden, in zunehmenden Maße direktziehende Haarfarbstoffe an Bedeutung. Direktziehende Farbstoffe bieten den Vorteil, daß sie ohne Zusatz von Oxidationsmitteln (beispielsweise Wasserstoffperoxid) zur Anwendung kommen und deshalb das Haar während des Färbevorganges wesentlich weniger geschädigt wird.

Gute Haarfärbemittel müssen eine Vielzahl von Anforderungen erfüllen. So müssen sie die gewünschten Farbnuancen in ausreichender Intensität ausbilden und gleichmäßig auf das Haar aufziehen, ohne die Kopfhaut zu stark anzufärben. Die erzeugten Haarfärbungen müssen weiterhin eine ausreichende Stabilität gegen Licht, Wärme, Schweiß, Haarreinigungsmittel und die bei der Dauerverformung der Haare verwendeten Chemikalien aufweisen sowie gegen die Einwirkung von sauren Mitteln und verdünnten Säuren stabil sein. Schließlich sollen diese Mittel in toxikologischer und dermatologischer Hinsicht unbedenklich sein.

Da eine gleichmäßige Haarfärbung vom Haaransatz bis in die Haarspitzen in der Regel mit Farbstoffen, die zu einer einzigen Verbindungsklasse gehören, nicht möglich ist, werden normalerweise Kombinationen von Farbstoffen aus mehreren verschiedenen Verbindungsklassen - beispielsweise Farbstoffkombinationen, welche neben Amino-, Diamino- und Hydroxyaminonitrobenzolderivaten auch Azo- und Anthrachinonfarbstoffe enthalten - verwendet.

Der bisher am häufigsten verwendete Azofarbstoff ist das 4'-Amino-4-[bis-(β-hydroxyethyl)amino]-azobenzol (DISPERSE BLACK 9). Ebenfalls ist es aus der DE-OS 35 34 885 bekannt, das 4'-Amino-2-methyl-4-[bis-(β-hydroxyethyl)amino]-azobenzol als Haarfarbstoff einzusetzen.

Obwohl das 4'-Amino-4-[bis-(β-hydroxyethyl)amino]-azobenzol häufig in Haarfärbemitteln eingesetzt wird, ist dieser Farbstoff in anwendungstechnischer Hinsicht nicht völlig zufriedenstellend. Zum einen besteht der Verdacht, daß diese Verbindung mutagen ist, und zum anderen besitzen die mit dieser Verbindung erzielten Haarfärbungen, ebenso wie die mit den aus der DE-OS 35 34 885 bekannten Haarfärbemitteln erzielten Haarfärbungen, nur eine geringe Stabilität gegenüber der Einwirkung von Säuren und sauren Zubereitungen.

Es bestand daher die Aufgabe, Haarfärbemittel mit einem Gehalt an einem Azofarbstoff zur Verfügung zu stellen, die eine gute physiologische Verträglichkeit besitzen und deren Färbungen gegen Sauren und saure Zubereitungen, wie zum Beispiel saure Haarspülungen oder Haarreinigungsmittel, saure Dauerwellmittel und Fixiermittel, stabil sind und deshalb eine dauerhaftere und über einen längeren Zeitraum gleichbleibende Färbung der Haare ermöglichen.

Es wurde nunmehr gefunden, daß durch ein Mittel zum Färben von Haaren mit einem Gehalt an einem 4'-Amino-2-halogen-4-[bis(β-hydroxyethyl)amino]-azobenzol die gestellte Aufgabe in hervorragender Weise gelöst wird.

Gegenstand der vorliegenden Erfindung ist daher ein Mittel zum Färben von Haaren mit einem Gehalt an für Haarfärbemittel üblichen Zusätzen, welches dadurch gekennzeichnet ist, daß es mindestens ein 4'-Amino-2-halogen-4-[bis-(β-hydroxyethyl)amino]-azobenzol der allgemeinen Formel (I)

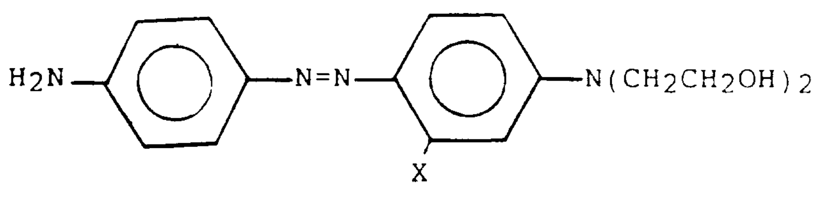

(I),

wobei X gleich Fluor, Chlor, oder Brom ist, enhält.

Von den erfindungsgemäßen Mitteln ist jenes bevorzugt, welches das 4'-Amino-2-chlor-4-[bis-(β-hydroxyethyl)amino]-azobenzol enthält. Die Farbstoffe der allgemeinen Formel (I) sollen in dem erfindungsgemäßen Mittel in einer Konzentration von 0,01 bis 2 Gewichtsprozent, vorzugsweise in einer Konzentration von 0,01 bis 1 Gewichtsprozent, enthalten sein.

Bei dem erfindungsgemäßen Haarfärbemittel handelt es sich um ein Mittel, das mindestens einen Farbstoff der allgemeinen Formel (I) enthält, oder aber um ein Mittel, das zusätzlich zu mindestens einem Farbstoff der allgemeinen Formel (I) noch einen oder mehrere weitere direkt auf das Haar aufziehende Farbstoffe enthält. Von diesen direkt auf das Haar aufziehenden Farbstoffen seien beispielsweise die folgenden erwähnt: aromatische Nitrofarbstoffe, wie zum Beispiel 2-Amino-6-chlor-4-nitrophenol, 2-Amino-4,6-dinitrophenol, 4-[(β-Hydroxyethyl)amino]-2-nitroanilin, 2-Chlor-6-ethylamino-4-nitro-benzol, $N^1$, $N^4$, $N^4$-Tris(β-hydroxyethyl)-p-phenylendiamin (HC BLUE 2), 4-[Bis-(β-hydroxyethyl)amino]-1-methylamino-2-nitrobenzol, 1-[(2,3-Dihydroxypropyl)amino]-4-ethyl-[(2-hydroxyethyl)amino]-2-nitrobenzol, 1-[(2,3-Dihydroxypropyl)amino]-4-dimethylamino-2-nitrobenzol, 1-[(2,3-Dihydroxypropyl)amino]-2-nitro-4-pyrrolidinobenzol, 4-[Bis-(β-hydroxyethyl)amino]-1-[(3-hydroxypropyl)amino]-2-nitrobenzol, 2-Amino-4-nitrophenol, 2-Nitro-1,4-diaminobenzol, 2-Amino-5-nitrophenol, 2-Amino-5-[(β-hydroxyethyl)amino]-nitrobenzol, 4-(β-Hydroxyethyl)amino-3-nitrophenol, 1-(β-Hydroxyethyl)amino-2-amino-4-nitrobenzol, 4-(β-Ureidoethyl)-amino-nitrobenzol, 4-(2',3'-Dihydroxypropyl)-amino-3-nitro-trifluormethylbenzol, 1,4-Bis-[(β-hydroxyethyl)-amino]-4-N-ethyl-2-nitrobenzol, 4-(β-Hydroxyethyl)-amino-3-nitrotoluol, 2,5-Bis-[(β-hydroxyethyl)amino]-nitrobenzol, 2-(β-Hydroxyethyl)amino-4,6-dinitrophenol, 1-Amino-4-(2',3'-dihydroxypropyl)amino-2-nitro-5-chlorbenzol, 1-Amino-2-nitro-4-[bis-(β-hydroxyethyl)amino]-benzol; Triphenylmethanfarbstoffe, wie zum Beispiel Basic Violet 1 (C.I. 42 535), Basic Violet 14 (C.I. 42 510), Basic Violet 2 (C.I. 42 520); Azofarbstoffe, wie beispielsweise Acid Brown 4 (C.I. 14 805); Anthrachinonfarbstoffe, wie zum Beispiel 1-[(β-Hydroxyethyl)amino]-4-methylamino-anthrachinon (DISPERSE BLUE 3; C.I. 61 505), DISPERSE BLUE 23 (C.I. 61 545), 1,4-Diamino-anthrachinon (DISPERSE VIOLET 1; C.I. 61 100), 1,4,5,8-Tetraaminoanthrachinon (C.I. 64 500), wobei die Farbstoffe dieser Klassen je nach Art ihrer Substituenten sauren, nichtionogenen oder basischen Charakter haben können. Weitere Geeignete, direkt auf das Haar aufziehende Farbstoffe sind beispielsweise in dem Buch von J. C. Johnson, "Hair Dyes", Noyes Data Corp., Park-Ridge (USA) (1973), Seiten 3-91 und 113-139 (ISBN: 0-8155-0477-2) beschrieben.

Der Gesamtgehalt an Haarfarbstoffen soll vorzugsweise 0,01 bis 3,0 Gewichtsprozent betragen.

Die Zubereitungsform des hier beschriebenen Haarfärbemittels, welches häufig auch als Tönungsmittel bezeichnet wird, kann beispielsweise die einer Lösung, insbesondere einer wäßrigen oder wäßrig-alkoholischen Lösung, sein. Bevorzugte Zubereitungsformen sind weiterhin die einer Creme, eines Geles, einer Emulsion oder die eines Schaumes, wobei sie auch im Gemisch mit einem Treibgas oder mittels einer Pumpe versprüht werden können.

Der pH-Wert dieses Färbemittels liegt im Bereich von 3 bis 12, insbesondere bei pH 8 bis 11,5, wobei die Einstellung des gewünschten alkalischen pH-Wertes hauptsächlich mit Ammoniak erfolgt, jedoch auch mit organischen Aminen wie beispielsweise Monoethanolamin oder Triethanolamin vorgenommen werden kann.

Die Verwendung des hier beschriebenen Haarfärbemittels erfolgt überlicherweise durch Aufbringen einer für die Haarfärbung ausreichenden Menge des Mittels auf die Haare, mit denen es etwa 5 bis 30 Minuten lang in Berührung bleibt. Anschließend wird mit Wasser, gegebenenfalls noch mit einer wäßrigen Lösung einer schwachen organischen Säure, gespült und sodann getrocknet.

Als schwache organische Säure können beispielsweise Essigsäure, Zitronensäure, Weinsäure und ähnliche Verwendung finden.

Das erfindungsgemäße Haarfärbemittel kann in Form eines Haarfärbemittels mit zusätzlicher Haarfestigung vorliegen, das mindestens ein in der Kosmetik üblicherweise verwendetes Polymerisat oder natürliches Polymer enthält. Solche Mittel werden im allgemeinen als Tönungsfestiger oder Farbfestiger bezeichnet.

Von den für diesen Zweck in der Kosmetik bekannten Polymerisaten seien beispielsweise Polyvinylpyrrolidon, Polyvinylacetat, Polyvinylalkohol oder Polyacrylverbingungen wie Polyacrylsäure beziehungsweise Polymethacrylsäure, basische Polymerisate der Ester der Polyacrylsäure oder Polymethracylsäure mit Aminoalkoholen beziehungsweise deren Salze oder Quaternisierungsprodukte, Polyacrylnitril, Polyvinyllactame sowie Copolymerisate aus diesen Verbindungen wie zum Beispiel Polyvinylpyrrolidon-Vinylacetat und dergleichen erwähnt.

Auch natürliche Polymere, wie zum Beispiel Chitosan (entacetyliertes Chitin) oder Chitosanderivate, können für den genannten Zweck eingesetzt werden.

Die Polymerisate und natürlichen Polymere sind in dem zuvor beschriebenen Haarfärbemittel in der für solche Mittel üblichen Menge von etwa 1 bis 5 Gewichtsprozent enthalten. Der pH-Wert des Mittels liegt im Bereich von etwa 6,0 bis 9,0. Die Anwendung dieses Haarfärbemittels mit zusätzlicher Festigung erfolgt in bekannter und üblicher Weise durch Befeuchten des Haares mit dem Festiger, Festlegen (Einlegen) des Haares zur Frisur und anschließende Trocknung.

Selbstverständlich kann das vorstehend beschriebene Haarfärbemittel gegebenenfalls weitere, für Haarfärbemittel übliche Zusätze, wie zum Beispiel Konservierungsstoffe und Parfümöle, Lösungsmittel wie Wasser, niedere aliphatische Alkohole, beispielsweise Ethanol, Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain, enthalten. Die erwähnten Bestandteile werden in den für solche Zwecke üblichen Mengen verwendet, zum Beispiel die Netzmittel und Emulgatoren in Konzentrationen von 0,5 bis 30 Gewichtsprozent, die Verdicker in einer Menge von 0,1 bis 25 Gewichtsprozent und die Pflegestoffe in einer Konzentration von 0,1 bis 5,0 Gewichtsprozent.

Die in den hier beschriebenen Haarfärbemitteln enthaltenen Verbindungen der Formel (I) sind toxikologisch und dermatologisch unbedenklich und ermöglichen Haarfärbungen mit einer hervorragenden Stabilität gegenüber Säuren und sauren Zubereitungen.

Hinsichtlich der färberischen Möglichkeiten bieten diese Haarfärbemittel je nach Art und Zusammensetzung der Farbkomponenten eine breite Palette verschiedener Farbnuancen, die sich von natürlichen Farbtönen bis hin zu hochmodischen, leuchtenden Nuancen erstreckt.

Gegenstand dieser Anmeldung sind weiterhin die neuen Verbindungen 4'-Amino-2-fluor-4-[bis-(β-hydroxyethyl)amino]-azobenzol und 4'-Amino-2-brom-4-[bis-(β-hydroxyethyl)amino]-azobenzol.

Diese neuen Verbindungen lassen sich, ebenso wie das 4'-Amino-2-chlor-4-[bis-(β-hydroxyethyl)amino]-azobenzol, durch ein zweistufiges Verfahren gemäß der folgenden Reaktionsgleichung herstellen (X = F, Cl, Br):

**1. Stufe:**

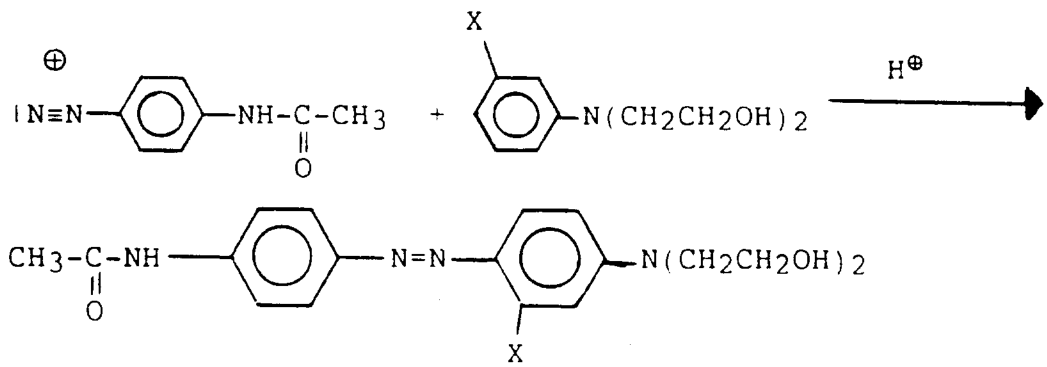

**2. Stufe:**

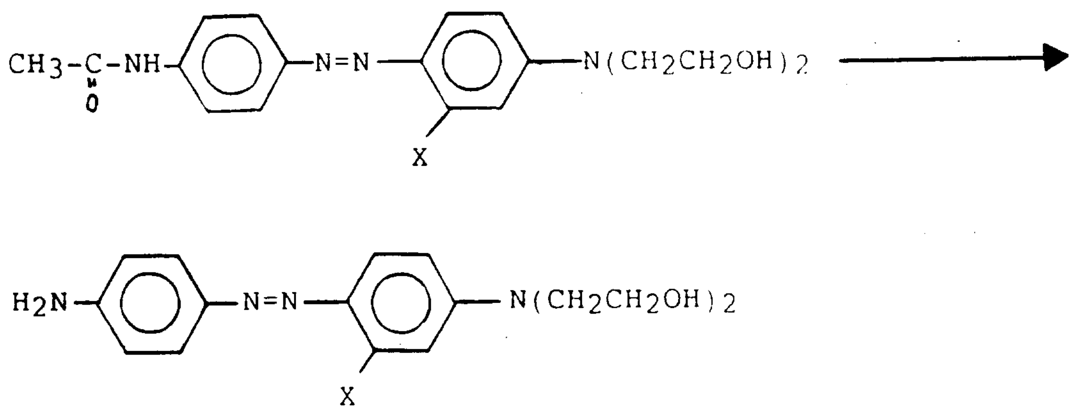

Das Herstellungsverfahren wird im einzelnen wie folgt durchgeführt:
Im Falle der Fluorverbindung wird zu einer wäßrigen Lösung des Diazoniumsalzes des p-Phenylendiamin-N-monoacetats in saurem Medium langsam unter Eiskühlung eine äquimolare Menge 3-Halogen-N,N-bis-(β-hydroxyethyl)-anilin getropft, während im Falle der Chlor- oder Bromverbindung die wäßrige Lösung des Diazoniumsalzes unter Eiskühlung zu einer äquimolaren Menge des 3-Halogen-N,N-bis-(β-hydroxyethyl)-anilins getropft wird. Die Reaktionsmischung wird sodann 24 Stunden lang bei Raumtemperatur gerührt.

Anschließend wird das Reaktionsprodukt entweder abfiltriert oder die Reaktionsmischung mit Diethylether extrahiert und der Etherextrakt im Vakuum eingedampft. Das erhaltene 4'-(Acetylamino)-2-halogen-4-[bis-(β-hydroxyethyl)amino]-azobenzol wird sodann in einer Mischung aus Ethanol und konzentrierter Salzsäure (Verhältnis Ethanol: HCl = 5 : 2) oder Ethanol und 2n Natronlauge (Verhältnis Ethanol: Natronlauge = 1 : 1) mehrere Stunden lang unter Rückfluß erhitzt. Sodann wird die Reaktionsmischung auf 0 Grad Celsius abgekühlt, mit Wasser verdünnt und mit einer 25-prozentigen Ammoniaklösung neutralisiert. Anschließend wird das erhaltene 4'-Amino-2-halogen-4-[bis-(β-hydroxyethyl)amino]-azobenzol mit Diethylether extrahiert, der Etherextrakt im Vakuum eingeengt und das erhaltene Produkt gegebenenfalls gereinigt.

Die nachfolgenden Beispiele sollen den Gegenstand der vorliegenden Erfindung näher erläutern, ohne ihn auf diese Beispiele zu beschränken.

**Herstellungsbeispiele**

**Beispiel 1:** Herstellung von 4'-Amino-2-fluor-4-[bis-(β-hydroxyethyl)amino]-azobenzol

**1. Stufe:** Herstellung von 4'-(Acetylamino)-2-fluor-4-[bis-(β-hydroxyethyl)amino]-azobenzol

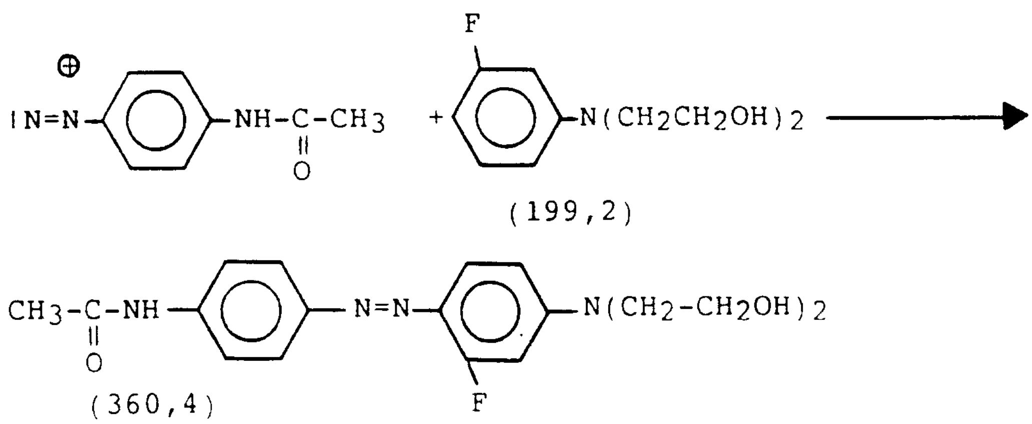

(199,2)

(360,4)

3 g (0,02 mol) p-Phenylendiamin-N-monoacetat werden in einer Mischung aus 10 g Wasser, 30 g zerstoßenem Eis und 3,4 ml konzentrierter Salzsäure mit 1,5 g (0,022 mol) Natriumnitrit bei O Grad Celsius diazotiert. Zu der erhaltenen Lösung des Diazoniumsalzes wird sodann innerhalb von zwei Stunden unter Rühren und Eiskühlung tropfenweise eine Lösung von 3,98 g (0,02 mol) 3-Fluor-N,N-bis-(β-hydroxyethyl)-anilin in 16 ml Wasser und 3,4 ml konzentrierter Salzsäure gegeben.

Anschließend wird die Reaktionsmischung 16 Stunden lang bei Raumtemperatur gerührt und sodann mit Ammoniak neutralisiert (pH = 7,5). Das Reaktionsprodukt fällt hierbei in Form eines dunkelbraunen Niederschlages aus. Es wird abfiltriert, mit Wasser gewaschen und über Calciumchlorid getrocknet.

Die Ausbeute beträgt 4,87 g (= 67,6 % der theoretischen Ausbeute).

| CHN-Analyse: ($C_{18}H_{21}FN_4O_3$) | | | |
|---|---|---|---|
| | % C | % H | % N |
| berechnet: | 59,99 | 5,87 | 15,55 |
| gefunden: | 58,62 | 5,74 | 15,10 |

**2. Stufe:** Herstellung von 4'-Amino-2-fluor-4-[bis-(β-hydroxyethyl)amino]-azobenzol

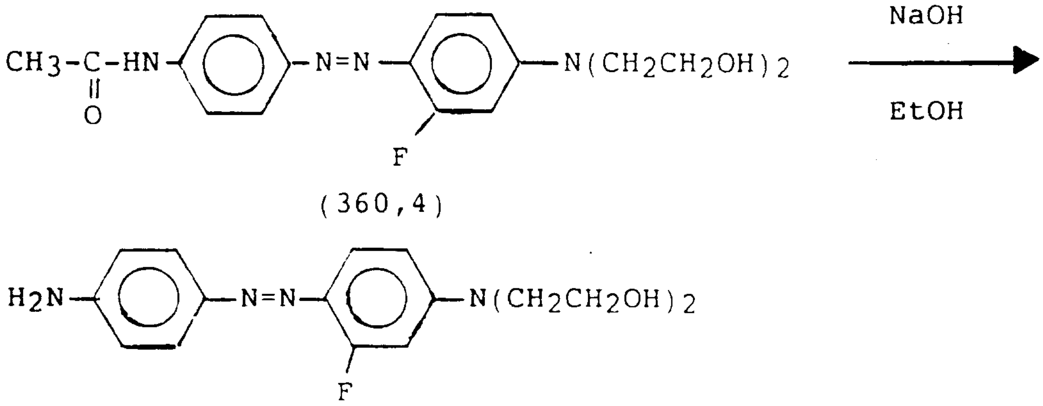


(360,4)

(318,4)

0,5 g (1,4 mmol) 4'-(Acetylamino)-2-fluor-4-[bis-(β-hydroxyethyl)amino]-azobenzol werden in einer Mischung aus 7,5 ml Ethanol und 7,5 ml 2n-Natronlauge 4 Stunden lang unter Rückfluß erhitzt. Anschließend wird die Reaktionsmischung auf 50 g zerstoßenes Eis gegossen, der orangefarbene Niederschlag abfiltriert, mit Wasser gewaschen und über Calciumchlorid getrocknet.

Die Ausbeute beträgt 0,35 g (= 78,5 % der theoretischen Ausbeute).

Der Schmelzpunkt beträgt 147 bis 149 Grad Celsius.

| CHN-Analyse: ($C_{16}H_{19}FN_4O_2$ x $H_2O$) | | | |
|---|---|---|---|
| | % C | % H | % N |
| berechnet: | 57,13 | 6,29 | 16,66 |
| gefunden: | 57,71 | 5,93 | 16,45 |

**Beispiel 2:** Herstellung von 4'-Amino-2-brom-4-[bis-(β-hydroxyethyl)amino]-azobenzol

**1. Stufe:** Herstellung von 4'-(Acetylamino)-2-brom-4-[bis-(β-hydroxyethyl)amino]-azobenzol

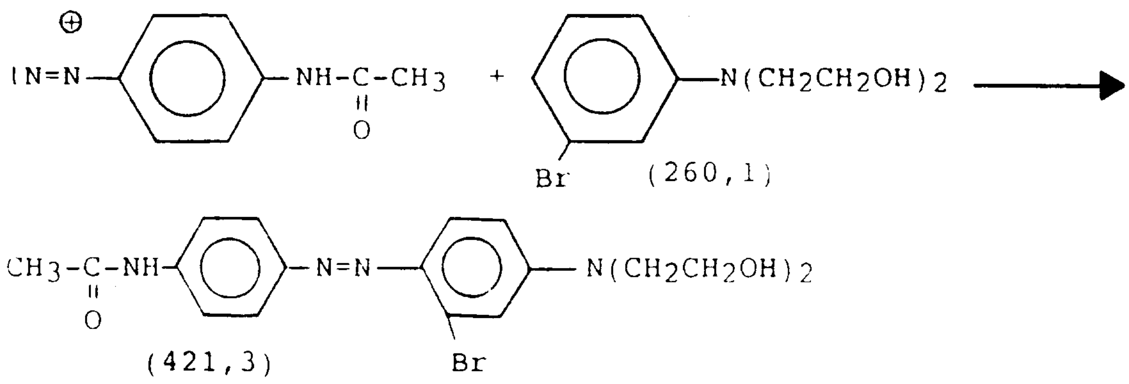


15 g (0,1 mol) p-Phenylendiamin-N-monoacetat werden in einer Mischung aus 50 g Wasser, 150 g zerstoßenem Eis und 17 ml konzentrierter Salzsäure mit 7,6 g (0,11 mol) Natriumnitrit bei 0 Grad Celsius diazotiert. Die erhaltene Lösung des Diazoniumsalzes wird filtriert und das Filtrat wird innerhalb von einer Stunde unter Rühren tropfenweise zu einer gekühlten Lösung von 26 g (0,1 mol) 3-Brom-N,N-bis-(β-hydroxyethyl)-anilin in 80 ml Wasser und 17 ml konzentrierter Salzsäure gegeben.

Anschließend wird die Reaktionsmischung 24 Stunden lang bei Raumtemperatur gerührt. Der ausgefallene Niederschlag wird sodann abfiltriert und im Exsikkator über Calciumchlorid getrocknet.

Es werden 9,5 g (= 20 % der theoretischen Ausbeute) 4'-(Acetylamino)-2-brom-4-[bis-(β-hydroxyethyl)amino]-azobenzol-Hydrochlorid-Hemihydrat erhalten.

Der Schmelzpunkt diese Produktes beträgt 187 bis 189 Grad Celsius (unter Zersetzung).

| CHNBrCl-Analyse: ($C_{18}H_{21}Br\ N_4O_3$ x HCl x 1/2 $H_2O$) | | | | | |
|---|---|---|---|---|---|
| | % C | % H | % N | % Br | % Cl |
| berechnet: | 46,28 | 4,53 | 12,00 | 16,94 | 7,61 |
| gefunden: | 46,60 | 4,80 | 10,80 | 17,10 | 7,90 |

**2. Stufe:** Herstellung von 4'-Amino-2-brom-4-[bis(β-hydroxyethyl)amino]-azobenzol

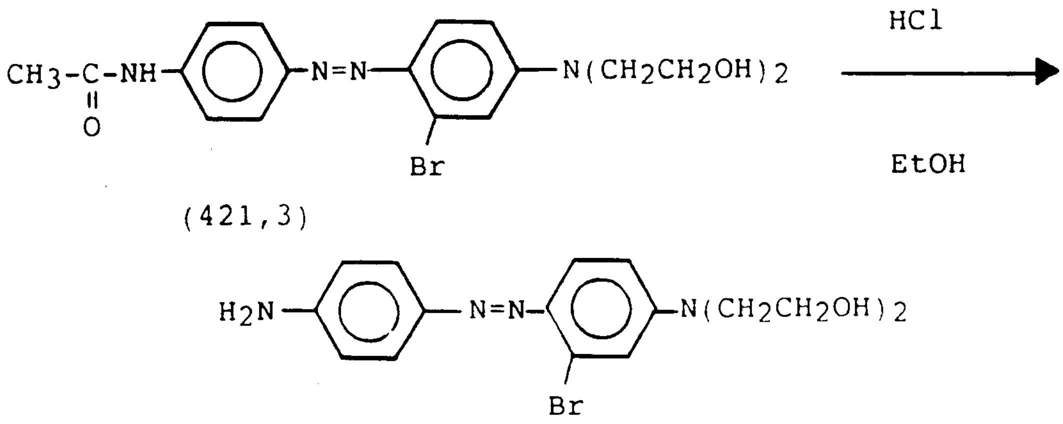


(421,3)

(379,3)

8,4 g (0,02 mol) 4'-Acetylamino-2-brom-4-[bis-(β-hydroxyethyl)amino]-azobenzol werden in einer Mischung aus 100 ml Ethanol und 40 ml konzentrierter Salzsäure 4 Stunden lang unter Rückfluß erhitzt. Anschießend wird die Reaktionsmischung auf 0 Grad Celsius abgekühlt, mit 200 ml Wasser vermischt und mit einer 25-prozentigen Ammoniaklösung neutralisiert.

Sodann wird die Lösung mit Diethylether extrahiert, der erhaltene Etherextrakt über Magnesiumsulfat getrocknet und filtriert. Nach dem Eindampfen des Etherextraktes werden 6,4 g (= 84 % der theoretischen Ausbeute) eines roten Öles erhalten, das langsam kristallisiert. Der Schmelzpunkt des kristallinen Produktes beträgt 120 bis 124 Grad Celsius.

| CHN-Analyse: ($C_{16}H_{19}Br\ N_4O_2$) | | | |
|---|---|---|---|
| | % C | % H | % N |
| berechnet: | 50,67 | 5,05 | 14,77 |
| gefunden: | 50,79 | 5,14 | 14,57 |

**Beispiel 3:** Herstellung von 4'-Amino-2-chlor-4-[bis-(β-hydroxyethyl)amino]-azobenzol

**1. Stufe:** Herstellung von 4'-(Acetylamino)-2-chlor-4-[bis-(β-hydroxyethyl)amino]-azobenzol

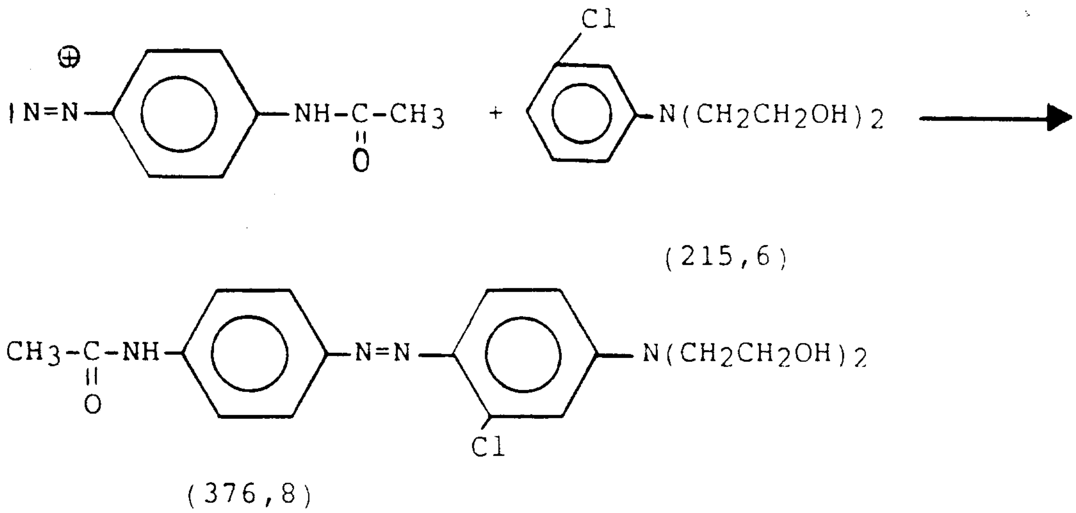


15 g (0,1 mol) p-Phenylendiamin-N-monoacetat werden in einer Mischung aus 50 g Wasser, 150 g zerstoßenem Eis und 17 ml konzentrierter Salzsäure mit 7,6 g (0,11 mol) Natriumnitrit bei 0° C diazotiert. Die erhaltene Lösung des Diazoniumsalzes wird filtriert und das Filtrat wird innerhalb von einer Stunde unter Rühren tropfenweise zu einer gekühlten Lösung von 21,6 g (0,1 mol) 3-Chlor-N,N-bis-(β-hydroxyethyl)-anilin in 80 ml Wasser und 17 ml konzentrierter Salzsäure gegeben.

Anschließend wird die Reaktionsmischung 24 Stunden lang bei Raumtemperatur gerührt. Sodann wird der ausgefallene Niederschlag abfiltriert und im Exsikkator über Calciumchlorid getrocknet.

Es werden 8,3 g (= 20 % der theoretischen Ausbeute) 4'-(Acetylamino)-2-chlor-4-[bis-(β-hydroxyethyl)amino]-azobenzol-Hydrochlorid erhalten.

Der Schmelzpunkt beträgt 192 Grad Celsius (unter Zersetzung).

**2. Stufe:** Herstellung von 4'-Amino-2-chlor-4-[bis-(β-hydroxyethyl)amino]-azobenzol

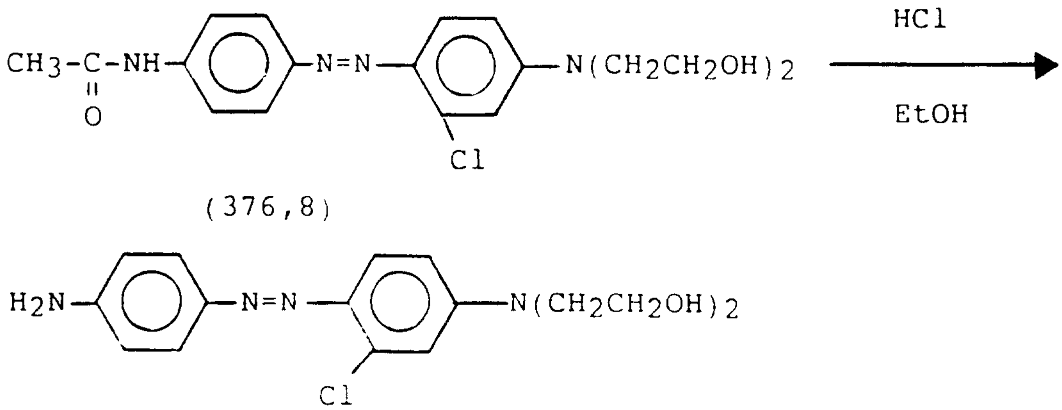


(376,8)

(334,8)

7,5 g (0,02 mol) 4'(Acetylamino)-2-chlor-4-[bis-(β-hydroxyethyl)amino]-azobenzol werden in einer Mischung aus 100 ml Ethanol und 40 ml konzentrierter Salzsäure 4 Stunden lang unter Rückfluß erhitzt.

Anschließend wird die Reaktionsmischung auf 0 Grad Celsius abgekühlt, mit 200 ml Wasser vermischt und mit einer 25-prozentigen Ammoniaklösung neutralisiert.

Sodann wird die Lösung mit Diethylether extrahiert, der erhaltene Etherextrakt über Magnesiumsulfat getrocknet und filtriert. Nach dem Eindampfen des Etherextraktes werden 5,2 g (= 77,6 % der theoretischen Ausbeute) eines roten Öles, welches langsam kristallisiert, erhalten.

Der Schmelzpunkt beträgt nach Umkristallisation in Toluol 146 bis 148 Grad Celsius.

| CHN-Anlage: ($C_{16}H_{19}Cl\ N_4O_2$) | | | |
|---|---|---|---|
| | % C | % H | % N |
| berechnet: | 57,40 | 5,72 | 16,73 |
| gefunden: | 57,55 | 5,78 | 16,68 |

**Beispiele für Haarfärbemittel:**

**Beispiel 4:** Haarfärbelösung

0,3 g 4'-Amino-2-halogen-4-[bis-(β-hydroxyethyl)amino]-azobenzol der allgemeinen Formel (I)
2,0 g Laurylalkoholdiglykolethersulfat-Natriumsalz (28-prozentige wäßrige Lösung)
2,0 g Ammoniak (25-prozentige wäßrige Lösung)
10,0 g Isopropanol
85,7 g Wasser
100,0 g

Gebleichtes Humanhaar wird 20 Minuten lang bei Raumtemperatur mit der vorstehenden Haarfärbelösung behandelt. Danach wird das Haar mit Wasser gespült und getrocknet. Das Haar ist wie in. nachfolgender Tabelle 1 angegeben

gefärbt.

Tabelle 1

| Haarfärbungen | |
|---|---|
| Verwendetes 4'-Amino-2-halogen-4-[bis-(β-hydroxyethyl)amino]-azobenzol | Haarfarbe |
| 4'-Amino-2-fluor-4-[bis-(β-hydroxyethyl)amino]-azobenzol | orange |
| 4'-Amino-2-chlor-4-[bis-(β-hydroxyethyl)amino]-azobenzol | orange |
| 4'-Amino-2-brom-4-[bis-(β-hydroxyethyl)amino]-azobenzol | intensiv orange |

**Beispiel 5:** Haarfärbecreme

0,04 g 4'-Amino-2-chlor-4-[bis(β-hydroxyethyl)amino]-azobenzol
0,30 g $N^1$,$N^4$,$N^4$-Tris-(β-hydroxyethyl)-2-nitro-p-phenylendiamin
0,03 g 1-Nitro-4-[(2-ureidoethyl)amino]-benzol
0,02 g Disperse Blue 3 (C.I. 61 505)
0,02 g Disperse Violet 1 (C.I. 61 100)
0,02 g 4-[(2-Hydroxyethyl)amino]-2-nitroanilin
7,00 g Cetylalkohol
2,00 g Laurylalkohol-diglykolethersulfat-Natriumsalz (28-prozentige wäßrige Lösung)
0,20 g Ammoniak (25-prozentige wäßrige Lösung)
0,10 g p-Hydroxybenzoesäuremethylester
90,27 g Wasser
100,00 g

50 g des vorstehenden Haarfärbemittels werden auf weiße menschliche Haare aufgetragen und nach einer Einwirkungszeit von 20 Minuten mit Wasser ausgespült.

Anschließend wird das Haar getrocknet. Das so behandelte Haar besitzt einen natürlichen Braunton.

**Beispiel 6:** Haarfärbelösung

0,05 g 4'-Amino-2-chlor-4-[bis-(β-hydroxyethyl)amino]-azobenzol
0,05 g 1,4-Diaminoanthrachinon
0,10 g $N^1$,$N^4$,$N^4$-Tris-(β-hydroxyethyl)-2-nitro-p-phenylendiamin
10,00 g Ammoniak (25-prozentige wäßrige Lösung)
5,00 g Laurylalkohol-diglykolethersulfat-Natriumsalz (28-prozentige wäßrige Lösung)
0,50 g Hydroxyethylcellulose
10,00 g Isopropanol
74,30 g Wasser
100,00 g

Gebleichtes menschliches Naturhaar wird 20 Minuten lang bei Raumtemperatur mit der vorstehenden Haarfärbelösung behandelt. Sodann werden die Haare mit Wasser gespült und mit einem Haarshampoo gewaschen. Nach dem Trocknen besitzen die Haare eine blond-aschfarbene Färbung.

**Beispiel 7:** Vergleichsversuche zur Säurebeständigkeit

Zum Vergleich der Säurestabilität der erfindungsgemäßen 4'-Amino-2-halogen-4-[bis-(β-hydroxyethyl)amino]-azobenzole mit aus dem Stand der Technik bekannten 4'-Amino-4-[bis-(β-hydroxyethyl)amino]-azobenzolen wurden Naturhaarsträhnen in der in Beispiel 4 beschriebenen Weise gefärbt, wobei folgende Haarfärbemittel verwendet wurden:

(i) ein Mittel gemäß Beispiel 4 (X = Cl),
(ii) ein Mittel gemäß Beispiel 4, in dem das erfindungsgemäße 4'-Amino-2-chlor-4-[bis-(β-hydroxyethyl)amino]-azobenzol durch die gleiche Menge an 4'-Amino-4-[bis-(β-hydroxyethyl)amino]-azobenzol ersetzt wurde,
(iii) ein Mittel gemäß Beispiel 4, in dem das erfindungsgemäße 4'-Amino-2-chlor-4-[bis-(β-hydroxyethyl)amino]-azobenzol durch die gleichet Menge an 4'-Amino-2-methyl-4-[bis-(β-hydroxyethyl)amino]-azobenzol ersetzt wurde,
(iv) ein Mittel gemäß Beispiel 4 (X = F).

Anschließend wurden die so gefärbten Haarsträhnen entweder

(A) mit 1n-Salzsäure eine Minute lang behandelt, sodann mit einem sauer eingestellten (pH=5,5) Shampoo gewaschen und getrocknet, oder

(B) mit einer mit 2n-Salzsäure auf pH 3 eingestellten Lösung von 10 g Natriumchlorid und 1 g Natriumdihydrogenphosphat in 1000 ml Wasser 4 Stunden lang behandelt, sodann mit Wasser ausgespült und getrocknet.

Die Lab-Farbwerte der erhaltenen gefärbten Haarsträhnen wurden mit einem Minolta-Farbmeßgerät, Typ CR-200 bestimmt. Die ermittelten Lab-Farbwerte sind in der nachfolgenden Tabelle 2 zusammengefaßt:

**Tabelle 2:** Farbmeßwerte-Waschversuche mit Säuren

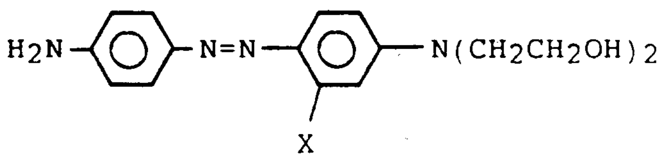

| | (A) | | (B) | |
|---|---|---|---|---|
| X | unbehandelt | 1n-Salzsäure | unbehandelt | Puffer pH=3 |
| (i) Cl | L 65,1 | 56,8 | 64,9 | 66,2 |
| | a 40,2 | 23,7 | 39,1 | 36,7 |
| | b 82,7 | 70,4 | 81,8 | 83,2 |
| (ii) H | L 58,8 | 37,5 | 66,8 | 64,4 |
| | a 41,7 | 6,0 | 33,1 | 25,1 |
| | b 77,3 | 38,1 | 85,0 | 79,7 |
| (iii) $CH_3$ | L 70,1 | 45,3 | 65,5 | 57,7 |
| | a 26,8 | -1,4 | 35,8 | 21,2 |
| | b 87,1 | 41,0 | 82,5 | 67,1 |
| (iv) F | L 69,2 | 68,8 | 70,1 | 69,7 |
| | a 24,8 | 19,7 | 24,2 | 19,8 |
| | b 82,4 | 76,3 | 84,2 | 80,3 |

Aus Tabelle 2 ist ersichtlich, daß die Farbmeßwerte der mit dem erfindungsgemäßem Mittel (i,iv) behandelten Haarsträhnen durch Behandlung mit Säuren in wesentlich geringerem Maße herabgesetzt werden als dies bei mit den bekannten Verbindungen (ii, iii) behandelten Haarsträhnen der Fall ist.

Die im Vergleich zum Stand der Technik wesentlich verbesserte Säurestabilität des erfindungsgemäßen Mittels wird noch deutlicher, wenn die Gesamtfarbänderung ΔY nach Anderson (vgl. US-PS 5 000 755) gemäß der folgenden Formel berechnet wird:

$$\Delta Y = \sqrt{(\Delta L)^2 + (\Delta a)^2 + (\Delta b)^2}$$

Die ermittelten Werte für die Gesamtfarbänderung ΔY sind in Tabelle 3 zusammengefaßt:

**Tabelle 3:** Gesamtfarbänderung ΔY

| 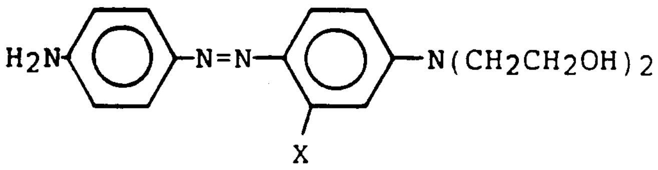 | | (I) |
|---|---|---|
| X | (A) 1n-Salzsäure | (B) Puffer pH=3 |
| (i) Cl | 22,2 | 3,1 |
| (ii) H | 57,1 | 9,9 |
| (iii) $CH_3$ | 59,5 | 22,6 |
| (iv) F | 8,0 | 5,9 |

Die mit dem erfindungsgemäßen Mittel (i, iv) behandelten Haarsträhnen zeigen bei der Einwirkung von 1n-Salzsäure eine wesentliche geringere Gesamtfarbändeung (ΔY) als die mit den bekannten Mitteln (ii, iii) behandelten Haarsträhnen.

Bei Behandlung mit einer sauren (pH=3) Pufferlösung bleiben die Farbmeßwerte für die mit dem erfindungsgemäßen Mittel (i, iv) behandelten Haarsträhnen nahezu unverändert, während die mit den bekannten Haarfärbemitteln (ii, iii) behandelten Haarsträhnen eine deutliche Farbänderung aufweisen.

Alle Prozentangaben stellen, soweit nicht anders vermerkt, Gewichtsprozente dar.

## Patentansprüche

1. Mittel zum Färben von Haaren mit einem Gehalt an für Haarfärbemittel üblichen Zusätzen, dadurch gekennzeichnet, daß es mindestens ein 4'-Amino-2-halogen-4-[bis-(β-hydroxyethyl)amino]-azobenzol der allgemeinen Formel (I)

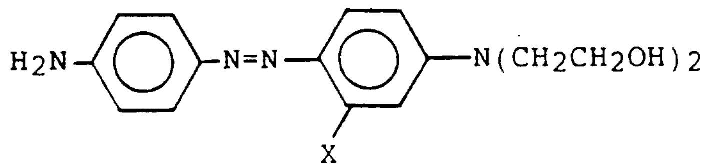

(I),

wobei X gleich Fluor, Chlor oder Brom ist, enthält.

2. Mittel nach Anspruch 1, dadurch gekennzeichnet, daß es 4'-Amino-2-chlor-4-[bis-(β-hydroxyethyl)amino]-azobenzol enthält.

3. Mittel nach Anspruch 1 oder 2, dadurch gekennzeichnet, daß es das 4'-Amino-2-halogen-4-[bis-(β-hydroxyethyl)amino]-azobenzol der Formel (I) in einer Menge von 0,01 bis 2 Gewichtsprozent enthält.

4. Mittel nach einem der Ansprüche 1 bis 3, dadurch gekennzeichnet, daß es einen pH-Wert von 3 bis 12 aufweist.

5. Mittel nach einem der Ansprüche 1 bis 4, dadurch gekennzeichnt, daß es in Form einer wäßrigen oder wäßrig-alkoholischen Lösung, einer Creme, eines Geles, einer Emulsion oder eines Schaumes vorliegt.

6. Mittel nach einem der Ansprüche 1 bis 5, dadurch gekennzeichnet, daß es zusätzlich einen oder mehrere direkt auf das Haar aufziehende Farbstoffe enthält.

7. Mittel nach Anspruch 6, dadurch gekennzeichnet, daß der zusätzlich enthaltene direkt auf das Haar aufziehende Farbstoff ausgewählt ist aus 2-Amino-6-chlor-4-nitrophenol, 2-Amino-4,6-dinitrophenol, 4-[(β-Hydroxyethyl)amino]-2-nitroanilin, 2-Chlor-6-ethylamino-4-nitro-benzol, $N^1$,$N^4$,$N^4$ -Tris(β-hydroxyethyl)-p-phenylendiamin , 4-[Bis-(β-hydroxyethyl)amino]-1-methylamino-2-nitro-benzol, 1-[(2,3-Dihydroxypropyl)amino]-4-ethyl-(2-hydroxyethyl)amino-2-nitrobenzol, 1-[(2,3-Dihydroxypropyl)amino]-4-dimethylamino-2-nitrobenzol, 1-[(2,3-Dihydroxypropyl)amino]-2-nitro-4-pyrrolidinobenzol, 4-[Bis-(β-hydroxyethyl)amino]-1-[(3-hydroxypropyl)amino]-2-nitrobenzol, 2-Amino-4-nitrophenol, 2-Nitro-1,4-diaminobenzol, 2-Amino-5-nitrophenol,2-Amino-5-[(β-hydroxyethyl)amino]-nitrobenzol, 4-(β-Hydroxyethyl)amino-3-nitrophenol, 1-(β-Hydroxyethyl)amino-2-amino-4-nitrobenzol, 4-(β-Ureidoethyl)-amino-nitrobenzol, 4-(2',3'-Dihydroxypropyl)amino-3nitrotrifluormethylbenzol, 1,4-Bis-[(β-hydroxyethyl)amino]-4-N-ethyl-2-nitrobenzol, 4-(β-Hydroxyethyl)-amino-3-nitrotoluol, 2,5-Bis-[(β-hydroxyethyl)amino]-nitrobenzol, 2-(β-Hydroxyethyl)amino-4,6-dinitrophenol, 1-Amino-4-(2',3'-dihydroxypropyl)amino-2-nitro-5-chlorbenzol, 1-Amino-2-nitro-4-[bis-(β-hydroxyethyl)amino]-benzol, Basic Violet 1 (C.I. 42 535), Basic Violet 14 (C.I. 42 510), Basic Violet 2 (C.I. 42 520), Acid Brown 4 (C.I. 14 805), 1-[(β-Hydroxyethyl)amino]-4-methylamino-anthrachinon, Disperse Blue 23 (C.I. 61 545), 1,4-Diamino-anthrachinon, 1,4,5,8-Tetraaminoanthrachinon.

8. Mittel nach einem der Ansprüche 1 bis 7, dadurch gekennzeichnet, daß es in Form eines Haarfärbemittels mit zusätzlicher Haarfestigung vorliegt und mindestens ein in der Kosmetik überlicherweise verwendetes Polymerisat oder natürliches Polymer enthält.

9. Mittel nach Anspruch 8, dadurch gekennzeichnet, daß das Polymerisat oder das natürliche Polymer ausgewählt ist aus Polyvinylpyrrolidon, Polyvinylacetat, Polyvinylalkohol, Polyacrylsäure, Polymethacrylsäure sowie basischen Polymerisaten der Ester der Polyacrylsäure oder Polymethacrylsäure mit Aminoalkoholen, deren Salzen oder Quaternisierungsprodukten, Polyarcylnitril, Polyvinyllactamen oder Copolymerisaten aus diesen Verbindungen, Chitosan und Chitosanderivaten.

10. 4'-Amino-2-fluor-4-[bis-(β-hydroxyethyl)amino]-azobenzol.

11. 4'-Amino-2-brom-4-[bis-(β-hydroxyethyl)amino]-azobenzol.

## Claims

1. Hair-colouring agent containing additives conventional for hair dyes, characterised in that it contains at least one 4'-amino-2-halogen-4-[bis-(β-hydroxyethyl)amino]-azobenzene of the general formula (I)

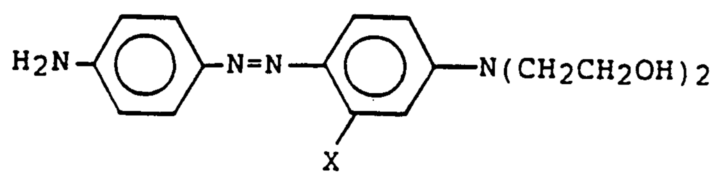

(I),

X being fluorine, chlorine or bromine.

2. Agent according to Claim 1, characterised in that it contains 4'-amino-2-chloro-4-[bis-(β-hydroxyethyl)-amino]-azobenzene.

3. Agent according to Claim 1 or 2, characterised in that it contains 4'-amino-2-halogen-4-[bis-(β-hydroxyethyl)amino]-azobenzene of formula (I) in an amount of from 0.01 to 2 weight %.

4. Agent according to any one of Claims 1 to 3, characterised in that it has a pH value of from 3 to 12.

5. Agent according to any one of Claims 1 to 4, characterised in that it is in the form of an aqueous or aqueous-alcoholic solution, a cream, a gel, an emulsion or a foam.

6. Agent according to any one of Claims 1 to 5, characterised in that it additionally contains one or a plurality of direct-acting hair dyes.

7. Agent according to Claim 6, characterised in that the additional, direct-acting dye is selected from 2-amino-6-chloro-4-nitrophenol, 2-amino-4,6-dinitrophenol, 4-[(β-hydroxyethyl)amino]-2-nitroaniline, 2-chloro-6-ethylamino-4-nitrobenzene, N1,N4,N4-tris(β-hydroxyethyl)-p-phenylenediamine, 4-[bis-(β-hydroxyethyl)amino]-1-methylamino-2-nitro-benzene, 1-[(2,3-dihydroxypropyl)amino]-4-ethyl-(2-hydroxyethyl)amino-2-nitrobenzene, 1-[(2,3-dihydroxypropyl)amino]-4-dimethylamino-2-nitrobenzene, 1-[(2,3-dihydroxypropyl)amino]-2-nitro-4-pyrrolidinobenzene, 4-[bis-(β-hydroxyethyl)amino]-1-[(3-hydroxypropyl)amino]-2-nitrobenzene, 2-amino-4-nitrophenol, 2-nitro-1,4-diaminobenzene, 2-amino-5-nitrophenol, 2-amino-5-[(β-hydroxyethyl)-amino]-nitrobenzene, 4-(β-hydroxyethyl)amino-3-nitrophenol, 1-(β-hydroxyethyl)amino-2-amino-4-nitrobenzene, 4-(β-ureidoethyl)-amino-nitrobenzene, 4-(2',3'-dihydroxypropyl)amino-3-nitrotrifluoromethylbenzene, 1,4-bis-[(β-hydroxyethyl)amino]-4-N-ethyl-2-nitrobenzene, 4-(β-hydroxyethyl)-amino-3-nitrotoluene, 2,5-bis-[(β-hydroxyethyl)amino]-nitrobenzene, 2-(β-hydroxyethyl)-amino-4,6-dinitrophenol, 1-amino-4-(2',3'-dihydroxypropyl)amino-2-nitro-5-chlorobenzene, 1-amino-2-nitro-4-[bis-(β-hydroxyethyl)amino]-benzene, Basic Violet 1 (C.I. 42 535), Basic Violet 14 (C.I. 42 510), Basic Violet 2 (C.I. 42 520), Acid Brown 4 (C.I. 14 805), 1-[(β-hydroxyethyl)amino]-4-methylamino-anthraquinone, Disperse Blue 23 (C.I. 61 545), 1,4-diamino-anthraquinone, 1,4,5,8-tetraamino-anthraquinone.

8. Agent according to any one of Claims 1 to 7, characterised in that it is in the form of a hair-colouring agent with an additional hair-setting agent and contains at least one natural polymer or polymerisate conventionally used in cosmetics.

9. Agent according to Claim 8, characterised in that the polymerisate or natural polymer is selected from polyvinyl pyrrolidone, polyvinyl acetate, polyvinyl alcohol, polyacrylic acid, polymethacrylic acid and basic polymerisates of polyacrylic acid or polymethacrylic acid esters with amino alcohols, their salts or products of quaternisation, polyacryl nitrile, polyvinyl lactams or copolymerisates of these compounds, chitosan and chitosan derivatives.

10. 4'-amino-2-fluoro-4-[bis-(β-hydroxyethyl)amino]-azobenzene.

11. 4'-amino-2-bromo-4-[bis-(β-hydroxyethyl)amino]-azobenzene.

## Revendications

1. Agent de teinture capillaire possédant une teneur en additifs usuels pour des agents de teinture capillaire, caractérisé en ce qu'il contient au moins un 4'-amino-2-haiogéno-4-[bis-(β-hydroxyéthyl)amino]-azo-benzène de formule générale (I)

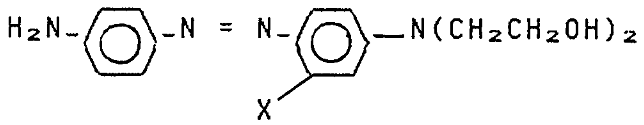

(I)

dans laquelle X représente le fluor, le chlore ou le brome.

2. Agent selon la revendication 1, caractérisé en ce qu'il contient du 4'-amino-2-chloro-4-[bis-(β-hydroxyéthyl)-amino]-azo-benzène.

3. Agent selon la revendication 1 ou 2, caractérisé en ce qu'il contient du 4'-amino-2-halogéno-4-[bis-(β-hydroxyéthyl)-amino]-azo-benzène de formule (I) à raison de 0,01 à 2% en poids.

4. Agent selon l'une des revendications 1 à 3, caractérisé en ce qu'il présente une valeur de pH allant de 3 à 12.

5. Agent selon l'une des revendications 1 à 4, caractérisé en qu'il se présente sous la forme d'une solution aqueuse ou hydro-alcoolique, d'une crème, d'un gel, d'une émulsion ou d'une mousse.

6. Agent selon l'une des revendications 1 à 5, caractérisé en ce qu'il contient additionnellement un ou plusieurs colorants se fixant directement sur les cheveux.

7. Agent selon la revendication 6, caractérisé en ce que le colorant se fixant additionnellement directement sur les cheveux est choisi parmi le 2-amino-6-chloro-4-nitro-phénol, le 2-amino-4,6-dinitro-phénol, la 4-[(β-hydroxyéthyl)amino]-2-nitro-aniline, le 2-chloro-6-éthylamino-4-nitro-benzène, la N1,N4,N4-tris(β-hydroxyéthyl)-p-phénylènediamine, le 4-[bis-(β-hydroxyéthyl)amino]-1-méthylamino-2-nitro-benzène, le 1-[(2,3-dihydroxypropyl)amino]-4-éthyl-(2-hydroxyéthyl) amino-2-nitro-benzène, le 1-[(2,3-dihydroxypropyl) amino]-4-diméthylamino-2-nitro-benzène, le 1-[(2,3-dihydroxypropyl)amino]-2-nitro-4-pyrrolidino-benzène, le 4-[bis-(β-hydroxyéthyl)amino]-1-[(3-hydroxypropyl) amino]-2-nitro-benzène, le 2-amino-4-nitro-phénol, le 2-nitro-1,4-diamino-benzène, le 2-amino-5-nitro-phénol, le 2-amino-5-[(β-hydroxyéthyl)amino]-nitro-benzène, le 4-(β-hydroxyéthyl)amino-3-nitro-phénol, le 1-(β-hydroxyéthyl)amino-2-amino-4-nitro-benzène, le 4-(β-uréidoéthyl)-amino-nitro-benzène, le 4-(2',3'-dihydroxypropyl)amino-3-nitro-trifluorométhyl-benzène, le 1,4-bis-[(β-hydroxyéthyl)-amino]-4-N-éthyl-2-nitrobenzène, le 4-(β-hydroxyéthyl)-amino-3-nitrotoluène, le 2,5-bis-[(β-hydroxyéthyl)amino-]-nitrobenzène, le 2-(β-hydroxyéthyl)-amino-4,6-dinitrophénol, le 1-amino-4-(2',3'-dihydroxypropyl)amino-2-nitro-5-chloro-benzène, le 1-amino-2-nitro-4-[bis-(β-hydroxyéthyl)amino]-benzène, le Basic Violet 1 (C.I. 42 535), le Basic Violet 14 (C.I. 42 510), le Basic Violet 2 (C.I. 42 520), l'Acid Brown 4 (C.I. 14 805), la 1-[(β-hydroxyéthyl)amino]-4-méthylamino-anthraqui-none, le Disperse Blue 23 (C.I. 61 545), la 1-4-diamino-anthraquinone, la 1,4,5,8-tétraamino-anthraqui-none.

8. Agent selon l'une des revendications 1 à 7, caractérisé en ce qu'il se présente sous forme d'un agent de teinture capillaire avec une fixation des cheveux additionnelle, et qu'il contient au moins un polymèrisat utilisé de manière usuelle dans les cosmétiques ou un polymère naturel.

9. Agent selon la revendication 8, caractérisé en ce que le polymèrisat ou le polymère naturel est choisi parmi la polyvinylpyrrolidone, l'acétate de polyvinyle, l'alcool polyvinylique, l'acide polyacrylique, l'acide polyméthacrylique, ainsi que les polymérisats basiques d'esters de l'acide polyacrylique ou polyméthacrylique avec des amino-alcools, leurs sels ou leurs produits de quaternisation, le polyarcylonitrile, les lactames de polyvinyle, ou les copolymères issus de ces composés, le chitosane et les dérivés du chitosane.

10. 4'-amino-2-fluoro-4-[bis-(β-hydroxyéthyl)-amino]-azo-benzène.

11. 4'-amino-2-bromo-4-[bis-(β-hydroxyéthyl)amino]-azo-benzène.